# EUROPEAN PATENT APPLICATION

(11) **EP 2 474 609 A1**
(43) Date of publication of application: **11.07.2012**
(21) Application number: 11150258.9
(22) Date of filing: 05.01.2011
(51) Int. Cl.: C12N 1/28, C12N 1/36

(54) **A method for the adaptive evolution of a cell and selection for a desired phenotype by serial propagation in an emulsion based system and uses thereof**

(71) Applicant: Stichting voor de Technische Wetenschappen, 3527 JP Utrecht (NL); Vereniging voor Christelijk Hoger Onderwijs, Wetenschappelijk Onderzoek en Patiëntenzorg, 1081 HV Amsterdam (NL)
(72) Inventor: Teusink, Bastiaan, 1081 HV Amsterdam (NL); Bachmann, Herwig, 1081 HV Amsterdam (NL)
(74) Representative: DeltaPatents B.V.

(57) **Abstract**

The invention provides a method for the adaptive evolution of a cell and selection for a desired phenotype by serial propagation in an emulsion based system whereby said desired phenotype is optionally; a) selection for high yield/biomass production b) increased or decreased production of primary and/or secondary metabolites c) and/or increased or decreased levels of macromolecules and d) increased levels of (genetically engineered) biotechnologically relevant products, and/or two or more different organisms that interact with each other.

## Description

### FIELD OF THE INVENTION

The invention is in the field of biology. In particular the invention relates to a method for the adaptive evolution of a cell and directed selection for a desired phenotype (phenotypic trait) by serial propagation in an *in-vitro* compartmentalized system, more in particular an emulsion based system whereby said desired phenotype is in particular but not limited to; a) selection for high yield/biomass production b) increased or decreased production of primary and/or secondary metabolites c) increased or decreased levels of macromolecules d) increased levels of (genetically engineered) biotechnologically relevant products e) selection for two or more cell(s)/bacterial types/species/organisms that may beneficially interact with each other to obtain a desired phenotype.

### BACKGROUND OF INVENTION

Growth is a fundamental property of life and that is highly optimized in unicellular organisms. In contrast to the situation for multi-cellular organisms, growth of biomass and reproduction are tightly coupled processes in uni-cellular organisms, since every cell division yields a new individual. This also applies to cells originating from multi-cellular organisms that have compromised growth control mechanisms, like tumor cells and cell lines. The rate of growth is an important factor in the long-term reproductive success of such cells. Mutants, which are able to regulate their cellular composition and metabolism in response to environmental conditions in a way that increases their growth rate under the given conditions, will outnumber their siblings. This is clearly the case under conditions of constant nutrient supply, but numerical dominance also provides an important fitness advantage under fluctuating nutrient supply, because it improves the odds for the mutant genotype to survive starvation and colonize new resources. This is the reason why mutants with higher growth rates than the parental strain can be obtained within a few hundred to thousand generations, a brief period on an evolutionary scale.

Literature suggests however, that faster growing microbes are more wasteful. It is well described for numerous organisms that high growth rates come at the cost of inefficient substrate utilization. An example is *Lactococcus lactis*, which under homolactic conditions converts glucose almost exclusively into lactate. An alternative metabolic strategy of *L. Lactis* would be the conversion of glucose into mainly acetate, formate, ethanol and CO₂. Such heterolactic fermentation would generate additional ATP and is therefore seen as metabolically more efficient. Increased metabolic efficiency is reflected in higher biomass yields, which is obtained with strains genetically engineered to be heterolactic.

The effect of this is that, on average, only 50% of the free-energy in growth media is converted into biomass during microbial growth. Thus microbial growth yield is approximately half of what would be expected based on the free-energy content of the supplied nutrients. This 50% loss of energy is described to be due to maintenance, overflow metabolism, futile cycles and growth-related but unidentified processes.

This growth-rate versus growth-yield trade-off has been difficult to assess experimentally. One reason is that current techniques for laboratory evolution result in the selection of the fastest growing strains (growth rate selection) and not in more efficiently but slower growing strains (yield-selection).

The latter has also practical consequences as metabolic engineering and (industrial) strain improvement are increasingly assisted by genome scale metabolic models such as Flux Balance Analysis (FBA) and OptKnock. However, these state-of-the-art computational modelling techniques are all based on growth-yield optimisation, not growth-rate optimization and it was demonstrated that this modelling approach tends to overestimate the biomass yield under conditions of excess nutrients, such as in batch growth of rapidly fermentable sugars.

In bacteria optimized growth rate and yield on several substrates is not necessarily a consequence of oxygen limitation during the experimental evolution, but a result of the actual conditions during the experimental evolution, which did not allow the selection of metabolically efficient, but slower growing strains. Because fast growing and metabolically inefficient strains are outcompeting metabolically efficient strains in suspension culturing systems it is thus currently impossible to select for cell(s)/cell culture/cell lines/strains/organisms with high metabolic efficiency at high substrate concentrations and therefore a desired phenotype like high biomass yield.

Selection for metabolic efficiency has not been possible to date with current methods, and hence metabolically efficient strains cannot be obtained without the use of metabolic engineering. Thus there is a need in the art for new targeted approaches to select for desired phenotypic traits/inherited characteristics. For example but not limited to increased biomass/yield through the direct selection of high yield cell types/strains without genetic manipulation as consumer acceptance currently limits the use of genetic engineering in industrially important sectors like fermentation (food, cheese, beer, yeast) and other industrially relevant biotechnology processes using living micro-organisms as 'cell factories' for industrial and manufacturing products and processes.

### SUMMARY OF THE INVENTION

The culturing and growing of microorganisms for industrial and laboratory purposes is mainly performed either in suspension cultures or cultures that have some sort of solid support structure such as agar plates. On agar plates, in a more structured environment, cells with different metabolic strategies hardly compete for the same resource, because they form relatively isolated colonies of genetically identical individuals using the energy efficient strategy. Under these conditions, selection on maximal biomass yield instead of growth rate will take place, because more biomass implies more individuals. However, population size is rather limited (relative low number of colonies), which makes it not suitable for the selection of desired mutant strains.

In suspension culturing systems cells are usually in close proximity to each other and substrates as well as fermentation products can diffuse freely between individual cells. Single-cells are therefore influenced by neighbouring cells, be it through the competitive consumption of substrates or the effect of secreted products. One of the intrinsic properties of suspension culturing systems is the fact that fast growing bacteria usually out-compete slower growing bacteria. Consequently, the prolonged propagation of microorganisms in suspension results in the selection of metabolically inefficient but fast growing bacteria, which outcompete metabolically efficient but slow growing bacteria.

An experimental approach to increase microbial yield - which in some cases comes with a decrease in unwanted by-product formation such as acetate - is by culturing organisms under carbon-limited conditions. However this strategy still does not allow the isolation of mutants with increased metabolic efficiency at high substrate concentrations.

Such problems were overcome by the present invention by the pioneering and innovative serial propagation technique where cells were cultivated in a water-in-oil emulsion, whereby the water phase was replaced by a culture medium suitable for the propagation of said cell. The sequential preparation and breaking of such emulsions by a method of the invention without compromising cell viability is a non described, challenging and not obvious procedure.

Surprisingly the serial propagation of cells in emulsion by a method of the invention by way of example showed that selective enrichment of a desired phenotype, the isolation of a cell (mutant cell) reaching a high biomass/yield compared to the ancestral cell could be readily obtained without the need for genetic engineering. In the examples genetically engineered strains were used as a proof of concept to demonstrate more clearly how quickly a strain with a desired phenotype like a high yield strain is lost in suspension cultures but in comparison is selectively enriched in the emulsion based system of the invention. However, it is understood by a person skilled in the art based on this information as disclosed herein the invention that a method of the invention will readily allow the selective enrichment of a cell (mutant cells) with desired phenotypic traits, like high biomass yield which are not genetically engineered in a similar manner. A trait is a distinct variant of a phenotypic character of an organism that may be inherited, environmentally determined or somewhere in between.

A selection method as disclosed herein the invention that allows the selection of both simple and more complex phenotypic traits like high biomass yield but not limited to, by experimental evolution in an emulsion based system without the need for the genetic modification of an organism opens lots of possibilities for future evolutionary engineering research, for example but not limited to, to study the molecular basis of evolved/desired phenotypes (phenotypic traits) for subsequent transfer to other hosts.

In the field of microfluidics it is not uncommon to prepare emulsions (also comprising cells) however published protocols concerning microdroplets are aimed at measuring certain parameters in droplets without the further propagation of them, for example characteristics used as a basis for identifying and/or separating micro-capsulated mutants cells. The serial propagation/sequential propagation of living cells in emulsion has not been described previously. Non obvious is the sequential preparation and breaking of emulsions without comprising bacterial viability. Most prior art publications teach the breaking of emulsions through the use of for example organic solvents or other amphiphilic molecules, which are often toxic for cells (Williams, Peisajovich, Miller, Magdassi, Tawfik, & Griffiths, 2006a; incorporated herein by reference).

The prior art teaches a method of breaking of emulsions and recovery of FACS sorted cells by plating on agar plates a procedure which is not suitable for prolonged serial propagation (Aharoni, Amitai, Bernath, Magdassi, & Tawfik, 2005; incorporated herein by reference). The serial propagation technique as described herein allows also more rapid, less costly and more simplified identification of a metabolically efficient mutant cell(s) even with only relatively small but commercially relevant increased biomass/yield increases than could not be identified using any techniques known in the art such as a FACS cell sorting method.

It is an object of the present invention to provide a novel and pioneering serial propagation method for the identification and isolation and selective enrichment of slow growing, metabolically efficient mutant strains with a desired phenotype like high biomass yield but not limited to, through experimental evolution in an emulsion based system without the need (but does not necessarily exclude the use of genetic engineering techniques) to use/resort to genetic engineering techniques. This pioneering technology has broad industrial and medical applications and will significantly extend the possibilities of adaptive/experimental evolution to address fundamental biological questions, like bacterial interactions and their influence on metabolic strategies and viability/culturability and the like.

### DETAILED DESCRIPTION OF THE INVENTION

Prior art selection procedures in organisms, because of technical limitation focus on properties which are usually related to a single (measurable) molecule, for example selection on the basis of a particular enzyme activity (Aharoni, Amitai, Bernath, Magdassi, & Tawfik, 2005; incorporated herein by reference) or the production of a desired measurable metabolite. In a preferred embodiment the invention provides a pioneering method for the selection of more complex phenotypic traits like cellular growth, yield/biomass production and the like. Surprisingly the serial propagation/emulsion based system as disclosed herein the invention allows for the first time the direct and simplified selection for (mutant) - cell(s), strain, culture, tissue, organism, cell types, species and the like with high metabolic efficiency and therefore high biomass/growth yield.

All terminology used herein the invention is well known in the art and described meanings encompassed herein. Mutant as used herein is an individual, organism, or new genetic character, arising or resulting from an instance of mutation either natural or induced/created. It includes any cell, strain, culture, tissue, organism, cell types, species, organism and the like differing from the wild type by whatever means. Mutants as used herein can be spontaneous or induced and includes loss of function mutants or gain of function mutants, which promotes traits that are preferably beneficial/desirable which have a positive effect on the phenotype and is a term known in the art.

Serial propagation as used herein refers to a propagation process which can be finite or continuous. Preferably continuous that is serial propagation that can be continued indefinitely.

(High) Biomass/yield as used herein for example can refer to; the ratio of the amount of biomass produced to the amount of substrate consumed (g biomass/g substrate), (a higher) number of actual cells produced, (increased) biological material, (higher) accumulation of living matter, a measure of (increased) output compared to wild type and the like but not limited to.

Strains as used herein can be cell strains adapted to culture with a finite or preferably continuous division potential. Organism as used herein can be a unicellular organism or a multicellular organism for example; whole/intact organisms, cell cultures/cell lines prepared directly from the organism or tissues of an organism adapted to living in suspension and the like.

Cell lines as used herein can be for example but not limited to; normal cell lines, hybrid cell lines, primary or secondary cell lines, stem cell lines and the like suitable for large scale production preferably with continuous division potential. Hybrid cell lines are constructed by fusing cells from different species and maintaining the resulting merged cells and their descendants in a culture medium.

Procedures for isolating cells (uniform and non-uniform) and growing them in culture are well known in the art. Cell or cell culture as used herein refers to cell(s) and culturing of cells derived from multicellular eukaryotes, example but not limited to animal cells (tissue and organ culture). But also includes cells and cultures of plants, fungi and microbes, including viruses, bacteriophages, bacteria and protists and the like. Cell culture and cell line includes both primary and secondary cell culture/lines.

It is understood that cell(s), mutant cell, strain, culture, tissue, (whole or part) organism, cell types, species and the like as disclosed herein the invention may and can be used interchangeably herein a method according to the invention where applicable and doable but are all terms with specific meaning well defined in the art.

In a preferred embodiment the invention provides a method for the adaptive/experimental evolution of a cell and selection for a desired phenotype by serial propagation in an emulsion based system. More in particular a method of the invention allows the selection and/or isolation of a mutant cell/strain achieving a delicate balance between metabolic efficiency and growth rate at high substrate concentrations thus optimally expressing a desired phenotype like high biomass yield and the like without the need for genetic engineering. For example but not limited to it favors the selection of a mutant cell/strain like a heterolactic cell/strain producing less undesirable energy inefficient wasteful products (like lactate) or potentially toxic side products (such as acetate or lactate) a common problem associated with outcompeting metabolically inefficient faster growing mutant strains.

In another embodiment said cell originates from a uni-cellular organism or self replicating/self propagating system known in the art, for example but not limited to a prokaryote organism. Preferably an industrially or medically relevant prokaryote organism *Escherichia coli, Bacillus sp.Propionibacterium sp. Streptomyces* and the like known in the art. The pioneering technology of the present invention paves the way to experimentally address fundamental questions, for example but not limited to like growth yield versus growth rate trade-off, thermodynamic efficiency or cell to cell interactions (or the absence thereof in an emulsion). For example how to optimise and/or manage microbial yield/growth rate trade-off (RC MacLean 2008; Heredity 100, 471 - 477; incorporated herein by referenence). It paves the way to improve metabolic efficiency of microorganisms without the use of genetic modification. It paves the way for the selection of strains with a decreased production of unwanted fermentation side products in industrial processes, like for example acetate production in microbial cultures reducing energy and material spillage (Eiteman & Altman, 2006: TRENDS in Biotechnology Vol.24 No. 11, p530: incorporated herein by reference).

In another embodiment said cell originates from a multi-cellular organism, a eukaryotic cell, for example but not limited to a cell with compromised growth control mechanisms like a tumor cell line and the like. The Warburg effect occurs in many types of tumor cells and in cell lines and neuro cells and is comparable to the metabolic inefficiency described in microbial metabolism and thus the technology can be easily adapted as such. For the purpose of the present invention the selection of slow growing, metabolically efficient cells can be considered at least equivalent to the selection of "healthy" cells , which might be of interest for a variety of challenges in the medical field known to a person skilled in the art.

Said cell may also originate from a plant like a plant protoplast, an algal cell, a fungal cell, a yeast cell and the like. A person skilled in the art can adapt the protocol, for example the growth/selection medium but not limited to in order to adapt/accommodate to each cell type. Thus a method as described herein the invention can be used in pioneering plant, algal, fungal, yeast and mammalian cell culture processes and the like for the elicitation of desired traits/wide-ranging products of biotechnology, industrial, and commercial uses, like for example secondary metabolites, alkaloids, industrial enzymes, therapeutic proteins, antibodies, vaccines, bioactive compounds, pharmaceuticals, feed, fine chemicals, renewable fuels and the like known in the art.

It is also understood that genetically engineered cells/strain/types can be used in a method of the invention. Conversely, cells may be genetically engineered after being optimized for metabolic efficiency.

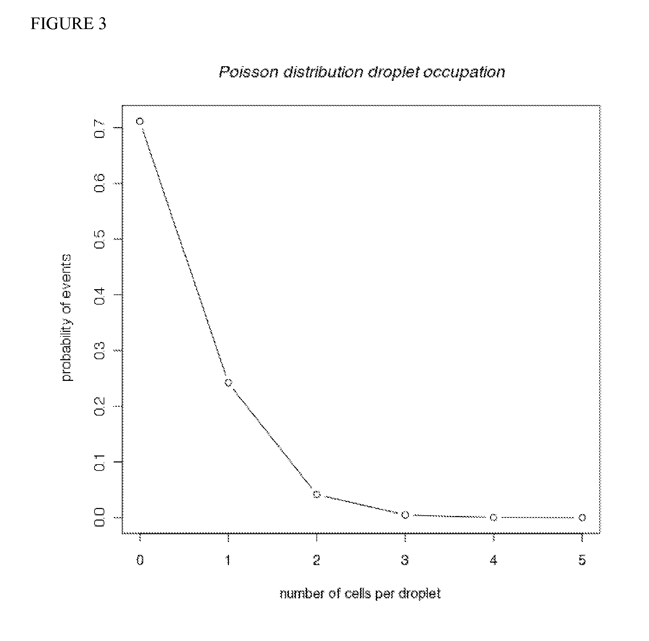

In another preferred embodiment said cell is compartmentalized in said emulsion based system in such a manner that direct competition for substrate or contact with other cells, like neighbouring cells is at least reduced/limited or eliminated without compromising on cell population size and/or substrate concentration and/or cell mutational frequency (mutational number/rate) and/or cell viability. Preferably a defined fraction of compartments/liquid droplets comprises a single cell derived from an individual organisms like an individual bacterial type/strain/species (see figure 3, Poisson distribution).

It is understood by a person skilled in the art that the frequency/rate of mutations can be increased by exposing the cells to mutagens, for example but not limited to UV, chemical mutagens, transposon induced mutations or the like, known in the art.

It is also understood by a person skilled in the art that any system where the cell is separated/compartmentalised/or contact is at least reduced from a neighbouring cell could be contemplated. The purpose of the oil emulsion is to compartmentalise each droplet comprising a single cell(s), to reduce/prevent contact with neighbouring droplets thus the competition for substrate. For example, any biotechnology system at least capable of separating (reducing contact between) individual droplets could be contemplated. Like microfluidic technologies, chip based systems (Ingham et al., 2007; incorporated herein by reference), or droplets (Zengler et al., 2002: incorporated herein by reference)(Aharoni, Amitai, Bernath, Magdassi, & Tawfik, 2005; incorporated herein by reference) or variations therefrom and the like which may allow compartmentalization, detection and subsequent isolation of a cell(s)/strains/organisms expressing a putative desired phenotype, which then may be scaled up and tested in the emulsion based system of the invention.

However, a method as disclosed herein the invention has a defined advantage over known in the art biotechnology systems. Because of known machine measuring errors (> 5%) known biotechnology methods/systems are increasingly limited when more fine/subtle phenotypic differences are sought which are industrially interesting and highly relevant. For example when the yield differences to be found is less than 5%, below the machine error threshold. A method as disclosed herein the invention is not prone to such problems because in an evolutionary trajectory using the emulsion system mutants with a higher yield will outperform the competition with time. Furthermore a method of the invention as disclosed herein is less expensive, can be scaled up for routine serial propagation of larger populations more applicable to large scale production in industrial and manufacturing and commercial biotechnological processes.

The oil phase of the emulsion is composed of any oil known in the art suitable for carrying out a method of the invention which does not compromise cell viability during the serial propagation process, preferably oxygen impermeable or permeable oils like mineral oil, fluorinated oil and the like. The water phase of the water-in oil emulsion (w/o) is formed by a growth medium at least suitable for the serial propagation and selective enrichment of said cell. The emulsion volume is prepared in such a way that individual droplets are inoculated with on average one cell per droplet (this will follow a Poisson distribution) and subsequently cells are allowed to grow in each droplet for a defined period of time, either to reach maximum cell density or at least a period of time which may be chosen as compromise between productivity, growth rate, cell number and the like.

The emulsion is broken, preferably by mechanical means like vortexing and/or stirring or any means that causes turbulence of the emulsion sufficient to cause physical separation of the liquid/oil emulsion mainly through physical stresses.

That mechanical means such as simple vortexing (without adding a solvent or any other chemical agents) was actually sufficient to break such an emulsion without compromising on cell viability was indeed surprising and unexpected as the prior art teaches that polydisperse emulsions are designed to be stable over long periods of time and are not that easy to break (Williams et al., 2006; incorporated herein by reference; Huebner *et al.,* 2008: Lab on a chip, incorporated herein by reference; Reagents *ABIL* EM *90*, a surfactant (Degussa) manufacturing instructions but not limited to.

Nor was it a trivial issue to maintain cell viability after breaking emulsions. Breaking the emulsion by mechanical means without resorting to organic solvents or other emulsion breaking agents (de-emulsifiers) known in the art had an unexpected positive effect on cell viability. Another aspect of culturing cells in emulsions was that the viability of (micro) organisms in the emulsion as disclosed herein the invention was found to be increased by more than two orders of magnitude compared to comparative suspension systems. A preferred organic compound tested that can be used in the breaking of an emulsion according to a method of the invention which did allow the recovery of viable cells was surprisingly petroleum-benzine (CAS: 101316-46-5). It is an object of the invention to provide a method according to the invention whereby said emulsion is broken using petroleum-benzine, and after serial propagation a desired mutant cell is identified and/or, selected and/or isolated.

After breaking the emulsion cells were recovered and the cell count established for example but not limited to; the number of colony forming units was established, for prokaryote systems up to 10⁶⁻10⁹ individual bacterial populations, preferably 10⁷ founded by single cells, can be cultured per milliliter culture medium, a suitable concentration to ensure that each liquid droplet comprises preferably a single cell for subsequent serial propagation of said cell in said emulsion based system. However, 10⁷ refers to a average droplet size of 40µm. If droplet size is reduced one might want more than 10⁷. A person skilled in the art can work out an objective range. For eukaryote systems also preferably 10⁶ to 10⁹ cells can be cultured per milliliter. However, like for bacterial systems this may be cell type dependent and a person skilled in the art could modify this accordingly.

Depending on the experimental setup the number of droplets and droplet diameter in the emulsion can be varied by changing the water to oil ratio in the emulsion and/or varying the surfactant concentration. An average droplet size of 10 µm would correspond to 1.91x10⁹ droplets per ml culture medium in the suspension and an average droplet size of 100 µm would correspond to 1.91x10⁶ droplets per ml. Any cell counting method known in the art that allows (rapid) cell enumeration can be employed in a method according to the invention, like flow cytometry, coulter counter and the like.

The process was repeated; a population of cells was serially propagated in said emulsion based system; the desired number of time sufficient to select for a mutant cell or cells/strain that reach the highest cell density in individual droplets irrespective of the time needed to grow to this cell density. Thus a method of the invention favours the selection of a cell or cells/strain based on metabolic efficiency rather than growth rate efficiency, or depending on incubation times between propagation steps - at least a cell which has reached the desired/optimal balance between yield/growth rate efficiency. Furthermore a method of the invention is suitable for implementation in large scale industrial commercial biotechnology industrial processes as a preferred replacement of large scale suspension cultures in said processes. The desired mutant cell/evolved cells/strain is identified, selected and isolated and characterised, preferably said desired mutant cell or cells/strain is a metabolically efficient cell or cells/strain that has reached the maximum cell density in said emulsion based system.

It is also understood that a method of the invention can be adapted to allow the propagation of a cell under aerobic conditions (using an oil phase which is able to transfer oxygen). This would broaden the usage of the invention to the serial propagation of cells derived from other organisms, cell types like *E. coli,* yeast and mammalian cell lines at various culture conditions.

In a preferred embodiment said cell is compartmentalized in said emulsion based system with at least a second cell or organism, for example one or more cells or organisms in such a manner that direct interaction with said second cell (one or more cells) or organism is achieved without compromising on cell population size and/or substrate concentration and/or cell mutational frequency and/or cell viability so that the presence or absence of interaction of said cell and said second cell can be evaluated. For example a droplet/compartment may comprise two or more different cell types, for example but not limited to two cells derived from one or more individual cell types/strains/species/organism (which may be present in/obtained/derived from complex mixtures, for example but not limited to complex environmental samples, mutant libraries, heterogeneous/homogenous cell populations, tissues, organism and the like) whose interaction may complement each other in a beneficial way for the production of a desired trait.

For example said cell or organism may interact beneficially and/or synergistically with said second cell/interacting partner cell or organism. For example an interacting partner cell set that is for example, one or more cells which interact optimally/beneficially "best-fit" (reciprocal self-recognition occurs between them) with each other for the production of a desired trait. Several examples for beneficial interactions are given in (Sieuwerts, de Bok, Jeroen Hugenholtz, & van Hylckama Vlieg, 2008; incorporated herein by reference). It is an object of the invention to provide use of a method according to the invention to enrich for a cell of interest and/or its interacting partner (cell(s), organisms), for example but not limited to from a complex mixture, for example complex environmental samples like soil, mutant libraries and the like (see section (e) as disclosed herein).

It is an object of the present invention to use a method of the invention to screen a high number of combinatorial possibilities of interacting organisms that is a large/high number and wide variety of interacting organisms, preferably beneficial interacting organisms present in complex mixtures.

For each cell type, volumes and parameters can be changed; other emulsion based systems can be tested using a method of the invention and adapted to achieve the same/similar results by a person skilled in the art.

In yet another preferred embodiment is a method according to the invention whereby said adaptive evolution of a cell; preferably but not limited to a bacterial cell; and selection for a desired phenotype by a serial propagation protocol of the invention for cellular growth/high yield/biomass production comprising the steps of;
a) Inoculating each droplet or at least a fraction of droplets of a liquid in oil (w/o) emulsion with a cell and/or a cell with an interacting partner cell or cell(s),
   whereby said liquid is a growth medium of a volume suitable for the serial propagation and selective enrichment of said cell. Suitable growth mediums are known in the art and also disclosed herein.
b) Incubating said emulsion for a defined/determined period of time, preferably until said cell has grown to a maximal cell density;
c) Breaking said emulsion, preferably by mechanical means vortexing or stirring, and/or using non-toxic chemical agents that do not impact on cell viability and the like, but not limited to;
d) Harvesting the suspension of grown cells, accurately enumerating cell density for example but not limited to through colony forming units, and then diluting said grown cells to a required concentration to ensure that each liquid droplet comprises preferably a single cell and/or a cell with an interacting partner cell or cell(s) (for example diluting said grown cells to a required concentration to ensure that a fraction of droplets comprises at least a defined (poisson determined) amount of cells).
e) Preparation of a new liquid in oil (w/o) emulsion and the process a)-d) is repeated,
f) Detection and/or isolation of a high biomass producing cell (mutant cell).

In a preferred embodiment of the invention the cells are accurately counted during the serial propagation procedure, for example cells are accurately enumerated after breaking the emulsion by using a Coulter-Counter to ensure the appropriate dilution of cells for subsequent serial propagation in the emulsion based system of the invention. Failing to enumerate cells accurately would in the case of overestimation lead to low inoculation densities and upon serial propagation to the total loss of cells. Underestimating the cell count would lead to too high inoculation densities and subsequently to a greater number of cells per droplet, in which case the selection of high yield spontaneous mutants may not work/be achieved anymore.

In another preferred embodiment using an emulsion based system as disclosed herein the invention up to 10⁶⁻10⁸ individual bacterial populations, preferably 10⁷ founded by single cells, can be cultured per millilitre, a suitable concentration to ensure that each liquid droplet comprises preferably a single cell for subsequent serial propagation of said cell in said emulsion based system. The compartmentalization in emulsions allows slow but metabolically efficient cells to reach higher cell densities within each droplet.

However, because the emulsion is polydisperse a fraction of the droplets do not contribute to the selection process - if droplets are too small they are not very likely to carry a cell, and if droplets are too big they are likely to carry more than 1 cell. For this reason the total volume of the emulsion has to be increased to reach the required population size. A volume suitable for the serial propagation and selective enrichment of said cell of the present invention is a volume of sufficient size to support a cell population size which allows sufficient mutations to occur, in order to identify arising mutants with high biomass yield. For example said volume is at least 30 ml but not limited to; a volume at least 2 to 3 orders of magnitude higher than that used by microfluidic systems known in the art.

Disclosed herein the invention is a pioneering method for the selection of (bacterial) strains/types/species with a high biomass yield, but not limited to. This selection is facilitated by a protocol that describes the propagation of bacterial populations in a water in oil (w/o) emulsion where the water phase of the emulsion is the growth medium of the organism. The emulsion is prepared in a way that on average each medium droplet is inoculated with one single cell and/or a cell with an interacting partner cell(s). Droplets are separated by the oil phase and no (or only very limited) diffusion processes between droplets are possible. The culture is incubated until cells in each droplet have grown to the maximum cell density or for a selected defined period of choice dependant on the result to be achieved. Because each droplet was inoculated preferentially with preferentially a single cell or a cell with an interacting partner cell(s) a slow growing but metabolically efficient cell/strain is not in competition with a fast growing cell/strain (thus the likelihood that a fast growing metabolically inefficient mutant evolves within one droplet is very low). This allows metabolically efficient cells to grow to higher cell densities in their respective droplets, as compared to the cell densities reached by metabolically inefficient cells. Once cells are fully-grown the emulsion is broken and cells are communally harvested (droplets are pooled). Because of the higher cell density reached in droplets by metabolically efficient cells their fraction in the harvested population will have increased by a factor determined by the difference in cell density reached by efficient/inefficient cells. The harvested cells are enumerated and subsequently used to prepare a new emulsion and the cycle is repeated as described above, herein the invention. In a method according to the invention a mutant cell with a subtle/otherwise non-detectable phenotypic difference like small increases in yield compared with the ancestor cell can be obtained compared with other known selection systems.

It is an object of the present invention to use a method according to the invention to perform the adaptive evolution of a cell(s) in an emulsion.

It is an object of the present invention to use a method according to the invention to increase the biomass yield of many industrial processes.

It is also an object of the present invention to use a method according to the invention for the stable propagation of metabolically efficient high-yield strains by reducing the risk of faster-growing metabolically inefficient mutants arising.

It is also an object of the present invention to use a method according to the invention to successfully propagate, identify and isolate a mutant cell with high metabolic efficiency expressing a desired phenotype like high biomass yield without the use of genetic engineering.

It is also an object of the present invention to use a method according to the invention to enrich for cell types/strains with a particular phenotype which could not be previously identified or isolated by known method in the art. To use a method according to the invention to create a new genetic diversity through the identification of rare mutations. Cell type as used herein can be for example eukaryotic or prokaryotic cell type, bacterial, mammalian, plant, yeast but not limited to these.

It is also an object of the present invention to use a method according to the invention for the selection of a desired phenotype; for example but not limited to
a) In biotechnology commercial, industrial and manufacturing processes; like fermentation processes but not limited to; for example to promote the growth of metabolic efficient bacterial strains producing a desired phenotype like high biomass yield.
b) In biotechnology commercial, industrial and manufacturing processes; like fermentation processes but not limited to; for example to reduce overflow metabolism, energy spill and production of unwanted (toxic) side products. Thus minimizing the production of unwanted fermentation side products, which are expensive to remove; e.g. acetate formation in *E. coli* fermentations (Eiteman & Altman, 2006; incorporated herein by reference). A method according to the invention can be used to clarify the influence of process parameters on growth, productivity and side product formation.
c) Medical processes for the selection of "healthy" slow growing mammalian cells in a fast growing population of mammalian tumor or neuronl cells. For example, the Warburg-effect describes rapidly growing tumor cells and cell lines that have a very high glycolytic rate and they produce lactate. In contrast the healthy wild type cells grow much slower but with a higher yield on glucose (McKeehan, 1982; Hsu and Sabatini, 2008; incorporated herein by reference).
d) Selection of cells/strains with increased production of primary or secondary metabolites, like antibiotics, toxins and the like. For example the production of cephalosporins in *Streptomyces clavuligerus* is described to be increased under conditions of nutrient limitation and the concomitant slow growth (Aharonowitz & Demain, 1978 *et al.,* incorporated herein by reference). This increased productivity is related to higher metabolic efficiency under the described conditions. Selection on the basis of metabolic efficiency as disclosed herein the invention would favor the increased production of secondary metabolites.
e) Identification of interactions between organisms. For example, but not limited to, selection for mutual beneficial interactions. A complex mixture of say for example but not limited to 1000s of different species/strains present in a sample of soil, the gastro intestinal tract, complex industrial starter cultures like cheese starter and the like, will be compartmentalized into droplets. If we generate e.g. 10⁸ droplets with 2 cells in each all possible combinations between 2 cells are present in these droplets. The number of 2-cell combinations scales with 0.5 n2, so with 1000 species there are 500,000 different combinations of species. With 10⁸ droplets each combination occurs 200 times. If in one droplet 2 cells show mutual beneficial interaction they will grow to higher cell densities/higher biomass/yield than cells in the other droplets and therefore they will be enriched using a method as described herein the invention. A method herein the invention provides a pioneering method indentify on a large scale mutual beneficial interaction between organisms, between cell and the like so in effect exploits extreme case of combinatorial possibilities. Several examples for beneficial interactions are given in (see Sieuwerts, de Bok, Jeroen Hugenholtz, & van Hylckama Vlieg, 2008: incorporated herein by reference).
f) Increasing bacteriocin production. The high ATP demand of bacteriocin production and a correlation of slow growth with increased bacteriocin production indicate that a method of the present invention would allow the selection of mutant strains with increased bacteriocin production (see De Vuyst, Callewaert, & Crabbe, 1996: incorporated herein by reference).
g) Selection procedures where the modification of (quorum) sensing parameters is required. For example for the selection of cells/strains with altered (quorum) sensing properties. This includes the sensing of environmental stimuli as well as stimuli from the same bacterial strain and stimuli derived from other organisms. (Diggle, Griffin, Campbell, & West, 2007: incorporated herein by reference; M Kleerebezem, Quadri, Kuipers, & W M de Vos, 1997: incorporated herein by reference.
h) Selection of cells/strains which synthesize and export novel or increased levels of macromolecules such as proteins, carbohydrates, lipids and mixed complexes thereof or (genetically engineered) biotechnologically relevant products. For example antibiotics or new targets for new antibiotics, vaccines and other therapies for infections.
i) Improvement to/and selection of cells/strains with high biotechnological relevance. Strain improvement mutant selection, to increase the production of the desired product. For example strain improvement for microorganisms like filamentous fungi (*Aspergillus niger, Mortierella alpine);* bacteria *Escherichia coli, Bacillus sp.Propionibacterium sp. Streptomyces sp):* Yeasts (*Saccharomyces cerevisiae, Kluyveromyces lactis, Pichia ciferrii, Phaffia rhodozyma*) and the like.
j) Stabilization of complex mixed cultures, which would normally be taken over by metabolically inefficient faster growing strains. This may be performed in combination with culturing steps from other systems known in the art, for example suspension systems.
k) Microorganisms experessing extracellular substrate degrading enzymes are often outcompeted by "cheating" mutants that do not express the extracellular enzyme but utilize the degradation products that were generated by the enzyme producing wild type strain. Examples are invertase, an extracellular yeast enzyme that degrades sucrose (Gore et al. 2009; doi:10.1038/nature07921; incorporated herein by reference) and an extracellular lactococcal protease, which degrades milk casein and is needed for efficient growth in milk (Bachmann et al. 2010; ISME Journal (2010), 1-4; incorporated herein by reference). Stability of the lactococcal protease is serious problem in the dairy industry. A method as disclosed herein the invention will allow for the enrichment of strains expressing the extracellular substrate degrading enzyme because competition between the wild type and invading cheaters is reduced/absent in the emulsion based system as described herein the invention. Extracellular enzymes are important for example but not limited to biofuel production, proteases produced for washing powders and the like.

It is an object of the present invention to provide a mutant cell/cell culture/cell lines/strains/organisms identified, selected and isolated by a method according to the invention. Preferably mutant cell/cell culture/cell lines/strains/organisms whereby said desired phenotype is optionally; the selection for a) for high yield/biomass production; b) for increased or decreased production of primary and/or secondary metabolites; c) increased or decreased levels of macromolecules, d) increased levels of (genetically engineered) biotechnologically relevant products e) stabilization of complex mixed cultures, which would normally be taken over by metabolically inefficient faster growing strains f) cells sensitive to toxins (enrichment of cells sensitive to toxins) produced by other cell(s), organisms, and the like in a population, for example mixed population and the like but not limited to.

The invention further provides a mutant cell for use in a biotechnologically relevant process, for the synthesis of a product preferably a biotechnologically relevant medicinal or industrial or manufacturing (for example pharmaceutical manufacturing) product. Suitable products are well known in the art. For example the use of a selected mutant cell for the increased synthesis/production of primary or secondary metabolites, other industrially relevant macromolecules, pharmaceutical products, a disease specific biomarker, biologics - biological products in medicine, biological medicinal product and the like.

Biologics as used herein can be composed of sugars, proteins, or nucleic acids or complex combinations of these substances, or may be living entities such as cells and tissues. Biologics are isolated from a variety of natural sources - human, animal, or microorganism. With regard to the nature of its active substance, a biological medicinal product can consist of entire microorganisms or mammalian cells or of nucleic acids or proteinaceous or polysaccharide component(s) originating from a microbial, animal, human or plant source. With regard to its mode of action, a biological medicinal product can be a therapeutic medicinal product, an immunological medicinal product, gene transfer materials, or cell therapy materials.

For example the use of a selected mutant cell for the decreased synthesis of a wasteful industrial product or other costly side products synthesized by microorganisms like acetate which are costly to remove in biotechnologically important process.

### MATERIALS AND MATERIAL

### Example 1

*L. lactis* NZ9010 is a lactate dehydrogenase (*ldh)* deletion mutant of strain NZ9000. In NZ9010 glycolysis is diverted from pyruvate towards formate, ethanol I and acetate formation which yields more ATP than lactate formation. NZ9010 can therefore grow to a higher cell density although it takes longer to reach the full cell density (Bongers et al., 2003; incorporated herein by reference).
- Under anaerobic conditions NZ9000 -> µ=0.78 and final OD=2.53
- Under anaerobic conditions NZ9010 -> µ=0.17 and final OD=3.14

*L. lactis* NZ9000 and *L. lactis* NZ9010 were cultured in a chemically defined medium (CDM), which was based on the medium described by (Poolman & Konings, 1988: incorporated herein by reference).
Medium composition:
- Glucose-monohydrate 24.98 mM, Potassium phosphate monobasic 20.21 mM, Sodium chloride 49.62 mM, Sodium phosphate dibasic 20.076 mM, DL-6,8-Thioctic acid 9.69 µM, D-Pantothenic acid hemicalcium salt 2.1 µM, Nicotinic acid 8.12 µM, Pyridoxal hydrochloride 4.91 µM, Pyridoxine hydrochloride (Pyridoxol.HCl) 4.86 µM, Thiamine hydrochloride 2.96 µM, Ammonium molybdate tetrahydrate 0.24 µM, Calcium chloride dihydrate 20.41 µM, Cobalt(II) sulfate heptahydrate 1.07 µM, Copper(II) sulfate pentahydrate 1.2 µM, Magnesium chloride hexahydrate 0.983 mM, Manganese chloride tetrahydrate 20.2 µM, Zinc sulfate heptahydrate 1.04 µM, Biotin 0.41 µM, L-Tyrosine 0.28 mM, L-Alanine 1.46 mM, L-Arginine 1.40 mM, L-Asparagine 0.61 mM, L-Aspartic acid 1.03 mM, L-Cysteine hydrochloride monohydrate 0.35 mM, L-Glutamic acid 0.66 mM, L-Glutamine 0.66 mM, Glycine 0.39 mM, L-Histidine 0.15 mM, L-Isoleucine 0.63 mM, L-Leucine 0.90 mM, L-Lysine monohydrochloride 1.02 mM, L-Methionine 0.25 mM, L-Phenylalanine 0.39 mM, L-Proline 3.58 mM, L-Serine 1.64 mM, L-Threonine 0.5709 mM, L-Tryptophan 0.18 mM, L-Valine 0.73 mM,

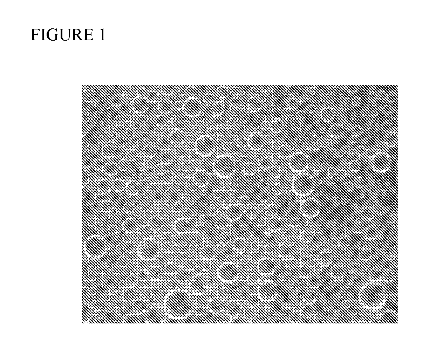

Cells were grown at 30°C over night to full cell density and subsequently NZ9000 and NZ9010 were mixed in a 70:30 ration respectively to a total cell density of 1x10⁷ cells per milliliter in CDM. Emulsions were prepared by mixing 3 ml of the diluted culture from above with 27 ml of mineral oil (Sigma) supplemented with 1.5% Abi190 (Degussa) in a 50 ml culture tube (GreinerBioOne #227261). Subsequently the tube was vortexed at 2300 rpm for 1 min, which lead to a polydisperse emulsion with an average droplet size of 35-40 µm in diameter. Droplet size was determined using laser diffraction measurements and light microscopy (see Figure 1).

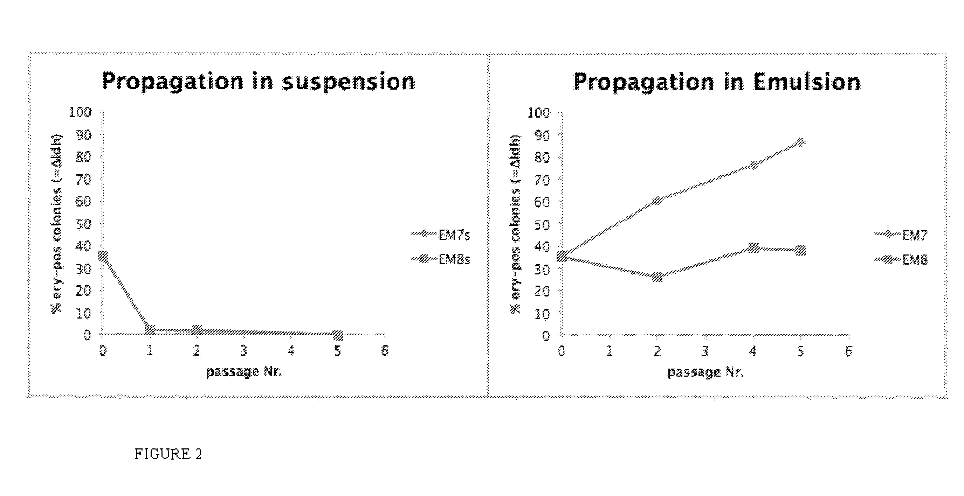

The emulsions were cultured for 2 days at 30°C to allow cells to reach full cell density in each inoculated droplet. During incubation the culture tubes were not completely closed to allow gas exchange, and they were kept in a jar filled with a nitrogen atmosphere. After two days 0.4 - 2 ml of emulsion (droplets were sedimented at the bottom of the tubes) were mixed with 10 ml of CDM and vortexed at 2300 rpm for 1 minute. Subsequently the tubes were centrifuged at 4500 g for 10 minutes and 60% of the supernatant was removed carefully. Cells were resuspended in the remaining medium and cells were counted using a Coulter Counter. Unexpectedly this protocol resulted in the recovery of 5x10⁷ to 7x10⁸ cells. The recovered cell suspension was diluted again to 10⁷ cells/ml and an emulsion was prepared as described above. This cycle was repeated 5 times. As controls the initial cell suspension (NZ9000:NZ9010 = 70:30) used for preparing the emulsion was also propagated in suspension cultures in 10 ml culture tubes. They were cultured at the same temperatures and culturing times as the emulsion. Propagation of the controls was done by diluting the cultures 1:100 into fresh CDM. Throughout the experiment dilutions of the control cultures and broken emulsions were plated on M17 medium (Oxoid) supplemented with 25 mM glucose and with and without 5 µg/ml erythromycin. Strain NZ9010 is erythromycin resistant (Bongers et al., 2003: incorporated herein by reference) and by dividing the number of colonies counted on plates containing erythromycin by the total number of cells as determined on plates without antibiotic the fraction of NZ9010 in the culture could be determined. The viability of cells recovered from emulsions with the described protocol was 100% as determined by a method of the invention. The results showed that the fraction of NZ9010 decreased rapidly if grown in the control cultures (in suspension) - see Figure 2. This propagation of a mixture of NZ9000 and NZ9010 in suspension shows rapid loss of NZ9010 (slow growing but high yield strain). However if cells were cultured in emulsion the fraction of NZ9010 was either stabilized or increased or enriched. Selection is accumulative and can detect smaller differences in yield, it is "just" a matter of time, because in an evolutionary trajectory using the emulsion system mutants with a higher yield will always succeed over the competition. Small yield differences are still industrially interesting and relevant.

In the examples genetically engineered strains were used as a proof of concept to demonstrate more clearly how quickly a strain with a desired phenotype like a high yield strain is lost in suspension cultures but in comparison is selectively enriched in the emulsion based system of the invention. However, it is understood by a person skilled in the art based on this information as disclosed herein the invention that a method of the invention will readily allow the selective enrichment of mutants with desired phenotypic traits, like high biomass yield which are not genetically engineered in a similar manner.

### Example 2:

EM7s and EM8s are duplicate experiments in suspension and EM7 and EM8 are duplicate experiments in emulsion. The viability of both strains did not differ significantly in emulsions. A droplet size of 40 µm corresponds to a droplet volume of 33.5 picoliter, which would give 2.98 x 10⁷ droplets per ml of medium used for emulsion preparation. Using a Poisson distribution one can calculate that on average roughly 1 in 4 droplets was inoculated with a single cell and that less than 5% of all droplets are occupied with 2 or more cells (Figure 3). Depending on the population composition more than one cell per droplet is likely to be a droplet co-occupation of strain NZ9010 and strain NZ9000. In such a case NZ9000 would out compete NZ9010 in that particular droplet. For this reason it is important to keep events of multiple occupation within droplets at a level acceptable for the experimental setup. The fact that one of the above propagation experiments resulted in stabilization rather than an increase in NZ9010 is probably caused by an inoculation density that was too high, showing the necessity for reducing multiple occupancy.

### Example 3. Breaking of the emulsion

Breaking the emulsion without compromising cell viability is one of the most critical steps during this protocol. Usually the breaking of emulsions for example emulsion PCR is preformed using e.g. organic solvents like diethyl-ether (Williams *et al.,* 2006: incorporated herein by reference). Using diethyl-ether for breaking the emulsions above resulted in no detectable viable cells. Furthermore other organic solvents as well as molecules that might interfere with the surfactant on the water/oil interface were tested for breaking the emulsion. The tested compounds included: petroleum, glycerol, Span80, Tween80, Triton-X100, octanol, hexanol and decanol. None of the listed compounds allowed the efficient breaking of the emulsion, thus showing that breaking emulsions whilst maintaining cell viability is neither obvious nor trivial. One tested organic compound that did surprising lead to the breaking of the emulsion and allowed the recovery of viable cells was petroleum-benzine (CAS: 101316-46-5). However the use of organic solvents to break emulsion may not be suitable for many applications as these agents themselves are potential carcinogens.

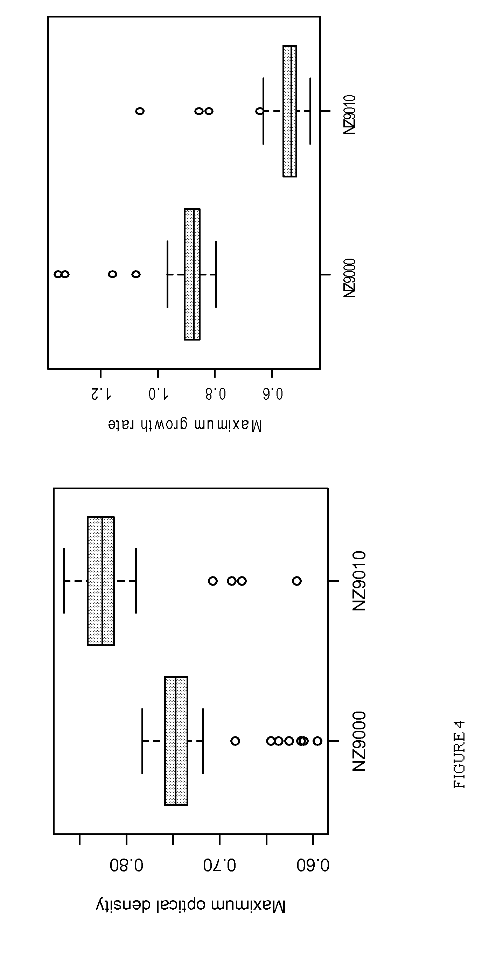

**Example 4**. Selection of desired phenotype on the basis of biomass yield measurements. In our example we used genetically engineered strains as a proof of concept. However it is understood by a person skilled in the art based on this information that a method of the invention will allow readily the selection of mutants with a desired phenotypic trait that are not genetically engineered. If the experimental evolution and selection for high yield is performed as described herein the invention single colonies originating from the evolved population can be picked and tested for the biomass yield in the desired conditions without the use of a biological marker. To shorten the experimental evolution this can be performed on a high throughput platform. For example throughout the evolution experiment single colonies can be isolated and inoculated into microtiterplates. The optical density is highly correlated to yield and it can be determined easily in a microplate reader (Figure 4).

### FIGURE LEGENDS

**Figure 1**: Polydisperse water in oil emulsion
**Figure 2**: Propagation of a mixture of *L. lactis* NZ9000 (fast growth but low yield) and NZ9010 (slow growth but high yield). During propagation in suspension (panel a) NZ9010 was lost rapidly whereas if the same mixture was propagated in emulsion (panel b) NZ9010 was enriched/stabilized.
**Figure 3**: Poisson distribution of droplet occupation. The distribution shows the probability (y-axis) of a certain number of cells per droplet (x-axis) occurring. The calculations were performed for a droplet size of 40 µm and a cell density of 1x10⁷ cells per ml in the suspension used to make the emulsion.
**Figure 4**: Maximum growth rate and maximum growth yield as determined for strains NZ9000 (n=96) and NZ9010 (n=96) in a 384-well microplate. Cells were grown in M17 (Oxoid) medium supplemented with 25 mM glucose at 30°C. Optical density at 600nm was measured in 10 minute intervals throughout the growth curve. NZ9000 has clearly a lower final optical density (lower yield) and a faster maximum growth rate. It demonstrates that strains with increased yield can be identified in a high throughput format.

### References

Aharoni, A., Amitai, G., Bernath, K., Magdassi, S., & Tawfik, D. S. (2005). High-throughput screening of enzyme libraries: thiolactonases evolved by fluorescence-activated sorting of single cells in emulsion compartments. Chemistry & biology, 12(12), 1281-9. doi: 10.1016/j.chembiol.2005.09.012.
Aharonowitz, Y., & Demain, A. L. (1978). Carbon catabolite regulation of cephalosporin production in Streptomyces clavuligerus. Antimicrobial agents and chemotherapy, 14(2), 159-64. Retrieved from http://www.pubmedcentral.nih.gov/articlerender.fcgi?artid=352426&tool=pmcentrez &rendertype=abstract.
Bongers, R. S., Hoefnagel, M. H. N., Starrenburg, M. J. C., Siemerink, M. A. J., Arends, J. G. A., Hugenholtz, J., et al. (2003). IS981-Mediated Adaptive Evolution Recovers Lactate Production by 1dhB Transcription Activation in a Lactate Dehydrogenase-Deficient Strain of Lactococcus lactis. Journal of bacteriology, 185(15), 4499-4507. doi: 10.1128/JB.185.15.4499-4507.2003.
De Vuyst, L., Callewaert, R., & Crabbe, K. (1996). Primary metabolite kinetics of bacteriocin biosynthesis by Lactobacillus amylovorus and evidence for stimulation of bacteriocin production under unfavourable growth conditions. Microbiology, 142(4), 817-827. doi: 10.1099/00221287-142-4-817.
Diggle, S. P., Griffin, A. S., Campbell, G. S., & West, S. a. (2007). Cooperation and conflict in quorum-sensing bacterial populations. Nature, 450(7168), 411-4. doi: 10.1038/nature06279.
Eiteman, M. a, & Altman, E. (2006). Overcoming acetate in Escherichia coli recombinant protein fermentations. Trends in biotechnology, 24(11), 530-6. doi: 10.1016/j.tibtech.2006.09.001.
Ingham, C. J., Sprenkels, A., Bomer, J., Molenaar, D., Berg, A. van den, Hylckama Vlieg, J. E. T. van, et al. (2007). The micro-Petri dish, a million-well growth chip for the culture and high-throughput screening of microorganisms. Proceedings of the National Academy of Sciences of the United States of America, 104(46), 18217-22. doi: 10.1073/pnas.0701693104.
Kleerebezem, M, Quadri, L. E., Kuipers, O. P., & Vos, W M de. (1997). Quorum sensing by peptide pheromones and two-component signal-transduction systems in Gram-positive bacteria. Molecular microbiology, 24(5), 895-904. Retrieved from http://www.ncbi.nlm.nih.gov/pubmed/9219998.
Poolman, B., & Konings, W. N. (1988). Relation of growth of Streptococcus lactis and Streptococcus cremoris to amino acid transport. Journal of bacteriology, 170(2), 700-7. Retrieved from http://www.pubmedcentral.nih.gov/articlerender.fcgi?artid=21071&tool=pmcentrez &rendertype=abstract.
Sieuwerts, S., Bok, F. A. M. de, Hugenholtz, Jeroen, & Hylckama Vlieg, J. E. T. van. (2008). Unraveling microbial interactions in food fermentations: from classical to genomics approaches. Applied and environmental microbiology, 74(16), 4997-5007. doi: 10.1128/AEM.00113-08.
Williams, R., Peisajovich, S. G., Miller, O. J., Magdassi, S., Tawfik, D. S., & Griffiths, A. D. (2006a). Amplification of complex gene libraries by emulsion PCR. Nature methods, 3(7), 545-50. doi: 10.1038/nmeth896.
Williams, R., Peisajovich, S. G., Miller, O. J., Magdassi, S., Tawfik, D. S., & Griffiths, A. D. (2006b). Amplification of complex gene libraries by emulsion PCR. Nature methods, 3(7), 545-50. doi: 10.1038/nmeth896.
Zengler, K., Toledo, G., Rappe, M., Elkins, J., Mathur, E. J., Short, J. M., et al. (2002). Cultivating the uncultured. Proceedings of the National Academy of Sciences of the United States of America, 99(24), 15681-6. doi: 10.1073/pnas.252630999.

## Claims

1. A method for the adaptive evolution of a cell and selection for a desired phenotype by serial propagation in an emulsion based system.

2. A method according to claim 1, whereby said cell originates from a uni-cellular organism.

3. A method according to claim 1, whereby said cell originates from a multi-cellular organism.

4. A method according to any of claims 1-3 whereby said cell is compartmentalized in said emulsion based system in such a manner that direct competition with neighbouring cells is reduced or eliminated without compromising on cell population size and/or substrate concentration and/or cell mutational frequency and/or cell viability.

5. A method according to any of claims 1-3 whereby said cell is compartmentalized in said emulsion based system with at least a second cell in such a manner that direct interaction with said second cell is achieved without compromising on cell population size and/or substrate concentration and/or cell mutational frequency and/or cell viability so that the presence or absence of interaction of said cell and said second cell can be evaluated.

6. A method according to any of claims 1-5, whereby said emulsion based system is a water-in-oil emulsion (w/o), whereby the water phase is replaced by a growth medium suitable for the serial propagation and selective enrichment of said cell.

7. A method according to claim 6 whereby said cell is subsequently serially propagated in said emulsion based system.

8. A method according to any of claims 4 - 7, whereby said emulsion is broken by mechanical means and a desired mutant cell is identified and/or selected and/or iso lated.

9. A method according to any of claims 4 - 7, whereby said emulsion is broken using petroleum-benzine and a desired mutant cell is identified and/or selected and/or isolated.

10. A method according to any of claim 1-9, whereby said adaptive evolution of a cell and selection for a desired phenotype by serial propagation is the selection for high yield/biomass production comprising the steps of;
a) Inoculating a fraction of droplets of a liquid in oil (w/o) emulsion with a cell, whereby said liquid is a growth medium suitable for the serial propagation and selective enrichment of said cell,
b) Incubating said emulsion until said cell has grown to a defined cell density,
c) Breaking said emulsion,
d) Harvesting said grown cells, enumerating cell count and diluting said grown cells to a required concentration to ensure that a fraction of droplets comprises preferably a single cell,
e) Preparation of a new liquid in oil (w/o) emulsion and the process a)-d) is repeated,
f) Detection and/or selection and/or isolation mutant cell/strain with a desired phenotype such as a metabolically efficient high biomass/yield producing mutant cell/strain.

11. A method according to any of claims 1-10, whereby said selection for a desired phenotype is the selection for high biomass/yield and/or increased or decreased production of primary and/or secondary metabolites and/or increased or decreased levels of macromolecules and/or increased levels of genetically engineered products and/or selection for cells sensitive to toxins produced by other organisms in a population.

12. Use of a method according to any of claims 5-11, to enrich for a cell of interest and/or its interacting partner from a complex mixture.

13. A mutant cell identified, selected and isolated by a method according to any of claims 1-11.

14. Use of a mutant cell according to claim 13 for the increased or decreased synthesis of a product.

15. Use according to claim 14, whereby said product is a biotechnologically relevant medicinal or industrial or manufacturing product.
